# EUROPÄISCHE PATENTSCHRIFT

(11) **EP 1 005 954 B1**
(45) Veröffentlichungstag und Bekanntmachung des Hinweises auf die Patenterteilung: **09.04.2003**
(21) Anmeldenummer: 99124124.1
(22) Anmeldetag: 02.12.1999
(51) Int. Cl.: B23Q 11/10, C02F 1/32

(54) **Vorrichtung zur Behandlung von Flüssigkeiten, insbesondere von Kühl- und Schmierstoffen**
Device for the treatment of liquids, especially of coolants and lubricants
Dispositif pour le traitement de liquides, en particulier de lubrifiants et de réfrigérants

(30) Priorität: 02.12.1998 DE 29821502 U
(43) Veröffentlichungstag der Anmeldung: 07.06.2000
(73) Patentinhaber: Metzger, Jürgen, 78532 Tuttlingen (DE)
(72) Erfinder: Metzger, Jürgen, 78532 Tuttlingen (DE)
(74) Vertreter: Patentanwälte Westphal, Mussgnug & Partner

(56) Entgegenhaltungen:
- EP-A- 0 444 530
- EP-A- 0 686 601
- DE-A- 19 515 710
- DE-C- 541 991
- FR-A- 2 450 612

## Beschreibung

Die Erfindung betrifft eine Vorrichtung zur Behandlung von Flüssigkeiten, insbesondere Kühl- und Schmierstoffen nach dem Oberbegriff des Anspruchs 1.

Bei der spanabhebenden Bearbeitung von Metallen, Glas, Keramik und so weiter sind vermehrt Kühl- und Schmierstoffe (nachfolgend KSS) genannt im Einsatz. Ein KSS dient insbesondere zur Kühlung des Werkzeugs und des Werkstücks, zur Schmierung und damit Herbsetzung der Reibungswärme sowie der Schnittkräfte und zur Reinigung des zu bearbeitenden Werkstücks beziehungsweise Werkzeuges durch Wegspülen der Späne und Verunreinigungen. Überwiegend werden wassermischbare KSS verwendet, die in Wasser emulgiert oder gelöst werden. KSS erfüllen dabei im Wesentlichen die Funktion des Schmierens, während das Wasser primär für die Kühlung verantwortlich ist. Im Sinne der vorliegenden Erfindung werden die Begriffe KSS und KSS-Emulsion austauschbar verwendet.

Beim Einsatz der KSS werden diese zunehmend mit Fremdstoffen belastet. Solche Fremdstoffe sind insbesondere Fremdöle, sowie Verschmutzung, die insbesondere von der spanabhebenden Bearbeitung herrühren. Weiter kann der KSS durch eigene Inhaltsstoffe belastet werden, die durch Zersetzungsprozesse entstehen. Schließlich kann der KSS auch durch Viren oder Mikroorganismen, wie Bakterien, Hefen und/oder Pilzen befallen werden, die zu einer biologischen Zersetzung und einer dadurch bedingten Untauglichkeit als KSS führen können. Regelmäßig ist mit der biologischen Zersetzung eine sehr unangenehme Geruchsbildung verbunden. KSS-Bäder können durch die Mikroorganismen auch in biologischer Hinsicht umkippen. Der Pilzbefall kann zu einem Zusetzen der KSS-Leitung führen, was langwierige und aufwendige Wartungs- und Reparaturarbeiten und damit kostspielige Stillstandzeiten zur Folge hat.

Um die Standzeit des KSS zu verlängern sind Maßnahmen bei handelsüblichen Vorrichtungen bekannt, die der Beseitigung dieser Fremdstoffe dienen. Feststoffverunreinigungen werden durch Abscheider und/oder Filter entfernt. Weiterhin ist bekannt, feine Luftblasen in den KSS einzubringen. Die feinen Luftblasen koppeln sich an Fremdstoffe an, wodurch sich deren Auftriebskraft wesentlich erhöht und eine schnellere Trennung der KSS von Fremdstoffen stattfindet. Durch das Einbringen der Luft kann es jedoch zu einer unerwünschten Anreicherung von aeroben Bakterien kommen, was das Problem der biologischen Zersetzung und des biologischen Kippens der KSS-Bäder verstärkt.

Auch in anderen Anwendungen, zum Beispiel Waschanlagen, Trinkwasserkreisläufen, und so weiter tritt häufig das Problem der biologischen Zusetzung oder Verunreinigung, des biologischen Umkippens und des Befalls mit Viren sowie Mikroorganismen, wie Pilzen und Bakterien auf.

Aus der FR-A-2450612 ist eine Vorrichtung gemäß dem Oberbegriff des Anspruchs 1 bekannt. Eine rotierende Trägertrommel taucht in eine mit dem flüssigen KSS gefüllte Wanne ein, wobei die Mantelfläche der Trägertrommel als Filmträger ausgebildet ist, der durch Adhäsion einen Flüssigkeitsfilm des KSS mitnimmt. In der Trägertrommel ist eine UV-Strahlungseinheit angeordnet, die durch den Mantel der Trägertrommel hindurch den Flüssigkeitsfilm bestrahlt. Ein Abstreifer trägt den Flüssigkeitsfilm nach der UV-Bestrahlung wieder von der Trägertrommel ab. Da der flüssige KSS durch Adhäsion von der rotierenden Trägertrommel mitgenommen wird, ist die Dicke des Flüssigkeitsfilms sehr stark von der Viskosität des KSS abhängig. Eine Beeinflussung und Optimierung der Dicke des Flüssigkeitsfilms ist nur sehr beschränkt möglich. Da die UV-Strahlung durch den Mantel der Trägertrommel hindurchtreten muß, ist die Materialauswahl für die Trägertrommel stark eingeschränkt. Die für die Bestrahlung des Flüssigkeitsfilms wirksame Intensität der UV-Strahlung wird beim Durchtritt durch die Trägertrommel abgeschwächt. Dabei wirkt sich insbesondere auch aus, dass die UV-Transparenz der Trägertrommel sich relativ schnell dadurch verschlechtert, dass sich auf der Oberfläche der Trägertrommel Schmutz absetzt und diese Oberfläche durch die sich in dem KSS befindenden abrasiven Partikel beschädigt und aufgeraut wird.

Aus der EP-A-0686601 ist eine UV-Behandlung von Trinkwasser bekannt, bei welcher das Trinkwasser über eine feststehende Fläche herabfließt und dabei mit UV-Licht bestrahlt wird.

Der Erfindung liegt die Aufgabe zugrunde, eine Vorrichtung zur Behandlung von Flüssigkeiten, insbesondere von Kühl- und Schmierstoffen, durch UV-Bestrahlung zu schaffen, die mit hoher Effektivität über lange Behandlungszeiträume wirksam ist.

Diese Aufgabe wird ausgehend von einem Stand der Technik der einleitend genannten Art durch die kennzeichnenden Merkmale des Anspruchs 1 gelöst.

Durch die in den Unteransprüchen genannten Maßnahmen sind vorteilhafte Ausführungen und Weiterbildungen der Erfindung möglich.

Dementsprechend zeichnet sich eine erfindungsgemäße Vorrichtung dadurch aus, dass eine Einrichtung zur Erzeugung eines Flüssigkeitsfilms und eine UV-Strahleinheit zur berührungslosen Bestrahlung des Films vorgesehen sind.

Die dünne Ausgestaltung des Films ermöglicht es, dass der Film von der UV-Strahlung vollständig durchdrungen wird und somit vollständig der desinfizierenden Wirkung der UV-Strahlung unterzogen wird. Durch die berührungslose Bestrahlung der Flüssigkeit bleibt die Oberfläche der UV-Strahleinheit frei von etwaigen Ablagerungen, die bei dem Eintauchen des UV-Strahlers in die Flüssigkeit, zum Beispiel in einen KSS, regelmäßig auftreten und die Oberfläche des UV-Strahlers gegenüber der gewünschten UV-Strahlung undurchlässig machen, so dass dieser ausgetauscht oder einer aufwendigen Reinigung unterzogen werden muß. Durch die berührungslose Bestrahlung des Flüssigkeitsfilms ergibt sich somit eine wesentlich längere Standzeit der UV-Strahlungseinheit.

Zwar ist die Wirkung der UV-Strahlung nicht von der Ausgestaltung eines durchgehenden flächigen Films abhängig, der Wirkungsgrad der erfindungsgemäßen Vorrichtung wird jedoch durch eine möglichst große bestrahlte Fläche bei zugleich ausreichend dünner Filmdicke erhöht, bei welcher Verunreinigungen oder Vorsprüngen nicht zum Auseinanderreißen des Flüssigkeitsfilms führen. Hierzu wird der Flüssigkeitsfilm auf einen beweglichen Filmträger aufgebracht Hierdurch wird die Fließgeschwindigkeit des Flüssigkeitsfilms auf dem Filmträger vermindert. Gegebenenfalls kann bei einer entsprechenden Abstimmung der Bewegung des Filmträgers eine relative Bewegung zwischen Filmträger und Flüssigkeitsfilm weitgehend unterbunden werden. In diesem Fall liegt ein stabiler flächiger Film im Bereich der UV-Strahlungseinheit vor, der gegebenenfalls in seiner Filmdicke definiert ausgebildet werden kann.

Als Filmträger ist eine rotierende Trommel vorgesehen, deren Mantelfläche als Tragfläche für den Film dient. Eine derartige Trägertrommel ist zum einen in der Herstellung sowie in ihrem Antrieb vergleichsweise wenig aufwendig. Darüber hinaus kann die als Tragfläche dienende Mantelfläche der Trägertrommel problemlos von außen her mit einer UV-Strahlungseinheit bestrahlt werden.

Dabei wird der Flüssigkeitsfilm im Bereich der UV-Strahlung in einer Abwärtsbewegung auf dem Filmträger befördert, so dass die Trägerbewegung mit der natürlichen Fließrichtung der Flüssigkeit richtungsmäßig übereinstimmt. Hierdurch kann, wie oben angeführt, die Relativbewegung zwischen dem Flüssigkeitsfilm und Filmträger erheblich reduziert werden.

Der-Filmträger wird von der Flüssigkeit mittels eines Gießkopfes angeströmt, bei dem die Flüssigkeit aus einem Füllvorrat über eine Ausströmöffnung oder Düse auf die Tragfläche des Filmträgers ausströmen kann. Dadurch wird die Flüssigkeit unmittelbar mit dem Auftreffen auf der Tragfläche des Filmträgers durch den Filmträger gefördert. Eine derartiger Gießkopf könnte anstelle einer Öffnung oder Düse auch einen Überlauf zur Filmbildung aufweisen.

In einer vorteilhaften Ausführungsform der Erfindung wird die Tragfläche mit einer Oberflächenstruktur versehen, durch die die Adhäsion der Flüssigkeit beziehungsweise die Filmbildung verbessert wird. Diese Oberflächenstruktur kann durch eine entsprechende Rauhigkeit oder Reliefstruktur gebildet werden. Denkbar ist jedoch auch, durch Beschichtung mit einem entsprechenden Material die Filmbildung zu verbessern.

Weiterhin kann die Oberfläche des Filmträgers derart ausgestaltet werden, dass sich eine Reflexionsfläche für die eingestrahlte UV-Strahlung ergibt. Hierdurch lässt sich der Wirkungsgrad der erfindungsgemäßen Vorrichtung deutlich erhöhen. Die Filmdicke kann hierbei so klein gewählt werden, dass die UV-Strahlung auch auf der Trägerseite mit einer noch deutlich wirksamen Intensität ankommt, an der Tragfläche reflektiert wird und somit in umgekehrter Laufrichtung erneut den Film durchdringt. Hierdurch ist eine homogenere Durchstrahlung des Films und damit eine effizientere Desinfektion der Flüssigkeit möglich.

Um die Flüssigkeit von dem Filmträger zu trennen, wird ein Abstreifer verwendet. Dadurch wird zuverlässig sichergestellt, dass die Flüssigkeit vollständig abgetragen und nicht etwa Reste auf der Tragfläche verbleiben, die die Menge der neu aufgenommenen Flüssigkeit beim erneuten Anströmen reduzieren würden. Weiterhin wird durch den Abstreifer die Tragfläche von eventuellen Verunreinigungen befreit. Der Abstreifer kann als statische Abstreiflippe angebracht werden. Weiterhin sorgt ein Abstreifer für einen definierten Abnahmeort der desinfizierten Flüssigkeit vom Filmträger, so dass ein entsprechender Auffangbehälter oder Trichter kleiner dimensioniert werden kann, als dies beispielsweise bei frei herabtropfender Flüssigkeit der Fall wäre.

Vorteilhafterweise wird die UV-Strahleinheit mit einem optischen Element zur Beeinflussung der Abstrahlcharakteristik versehen. In Frage kommt hierbei beispielsweise ein Parabolspiegel, der die aus dem UV-Strahler in Richtung von dem Flüssigkeitsfilm wegweisende Strahlung auf den Flüssigkeitsfilm spiegelt. Die Ausführung als Parabolspiegel ergibt hierbei einen nahezu parallelen Strahlengang der reflektierenden Strahlung. Durch derartige optische Elemente kann wiederum der Wirkungsgrad der erfindungsgemäßen Vorrichtung gesteigert werden, wodurch gegebenenfalls weniger oder kostengünstigere UV-Strahler zum Einsatz kommen können.

In einer Weiterbildung der Erfindung werden hierbei mehrere UV-Strahler vorgesehen, die gegebenenfalls alle optische Elemente zur Beeinflussung der Strahlungscharakteristik aufweisen können. Hierdurch lässt sich eine flächigere Bestrahlung erzielen. Die Größe der bestrahlten Fläche kann dabei der Filmdicke, der Fördergeschwindigkeit des Films vor dem UV-Strahler sowie dem entsprechenden Keimbefall derart angepasst werden, dass eine ausreichende gewünschte Desinfektion stattfindet.

Vorteilhafterweise wird weiterhin die Menge der vor der UV-Strahlungseinheit vorbeigeführten Flüssigkeit geregelt. Dies ist beispielsweise durch eine Regelung der Filmdicke möglich. Die Filmdicke wiederum kann beispielsweise durch entsprechende Querschnitte der Anströmöffnungen oder Düsen und/oder durch den die Anströmung verursachenden Druck der Flüssigkeit eingestellt werden. Der Druck der Flüssigkeit wiederum kann beispielsweise durch Regelung des Förderdrucks einer Förderpumpe variiert werden. Hierbei können auch Dosierventile, beispielsweise in Form von Regeldrosseln oder getakteten Ventilen, zum Einsatz kommen.

Für eine möglichst gleichmäßige Anströmung über die gesamte Filmbreite hat sich jedoch bewährt, einen Füllbehälter oberhalb der Ausströmöffnung bzw. Düse vorzusehen. In diesem Fall kann die Mengenregulierung der Flüssigkeit durch Regelung der Füllstandshöhe vorgenommen werden. Hierbei kommen beispielsweise elektronische Regelungen in Frage, die eine Füllstandsmessung in Verbindung mit einem entsprechenden Dosierventil vorsehen. Andere Ausbildungen sehen einen höhenverstellbaren Überlauf vor. Um die Fließmenge über einen solchen Überlauf unabhängig von der Durchflussmenge in der Zuleitung auszugestalten, kann beispielsweise der Überlauf in einer Bypassleitung zur Flüssigkeits-Zufuhr des Füllbehälters vorgenommen werden, die von dem Zufluss des Füllbehälters abzweigt und in den üblicherweise bei entsprechenden spanabhebenden Maschinen vorhandenen Vorratstank mündet. Eine weitere Möglichkeit, den Füllstand in einem Füllbehälter zu regulieren, besteht in Form eines Schwimmerventils:

Ein Ausführungsbeispiel der Erfindung ist in der Zeichnung dargestellt und wird anhand der Figuren nachfolgend beschrieben.

Im Einzelnen zeigen
- Fig. 1: eine schematische Seitenansicht einer erfindungsgemäßen Vorrichtung und
- Fig. 2: eine Ausschnittsvergrößerung eines Gießkopfs einer erfindungsgemäßen Vorrichtung gemäß Figur 1.

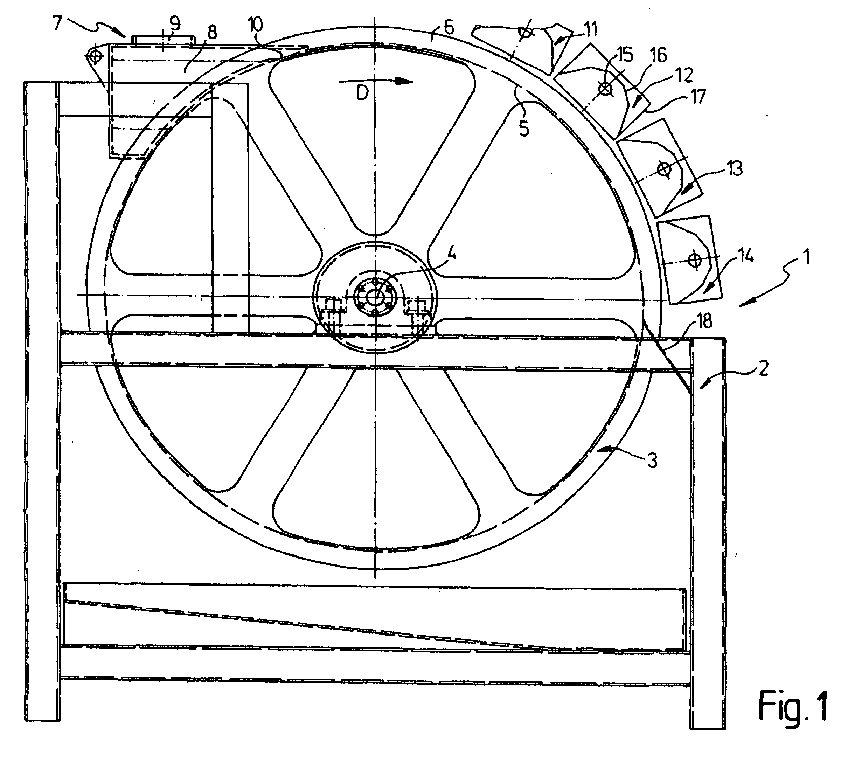

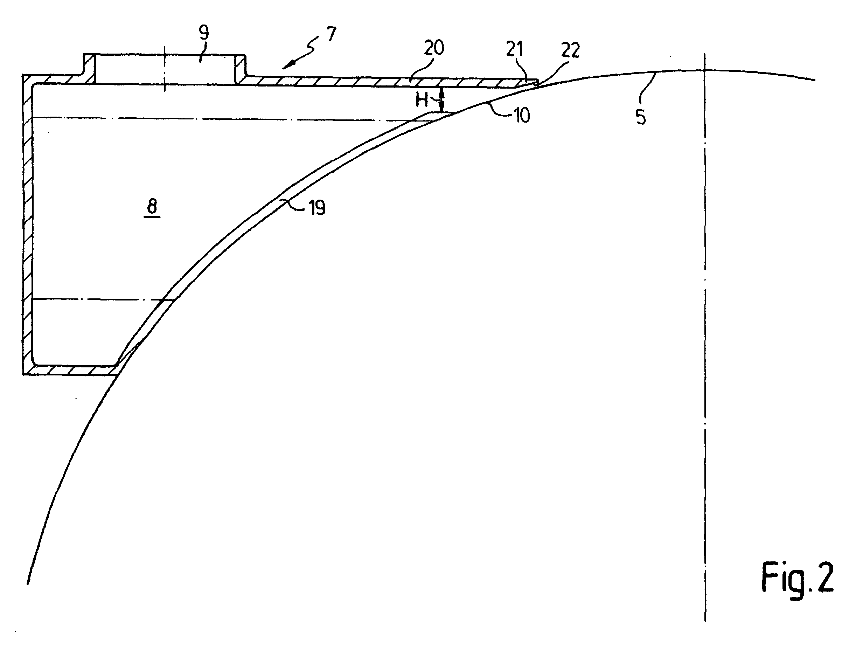

Die Vorrichtung 1 gemäß Figur 1 umfasst ein Rahmengestell 2, auf dem eine Trägertrommel 3 um eine Drehachse 4 drehbar gelagert ist. Die Trägertrommel 3 umfasst eine Tragfläche 5, die als Zylindermantelfläche ausgestaltet ist und die stirnseitig über Randflansche 6 begrenzt ist. Ein Gießkopf 7, bestehend aus einem Vorratsbehälter 8 (vgl. insbesondere Figur 2), einer Einfüllöffnung 9 sowie einer Ausströmöffnung 10 ist im oberen Bereich der Trägertrommel 3 angeordnet. Bezüglich der Drehrichtung D nachfolgend sind vier UV-Strahlungseinheiten 11, 12, 13, 14 entlang der Tragfläche 5 angeordnet.

Jede UV-Strahlungseinheit 11, 12, 13, 14 umfasst einen UV-Strahler 15, der im Brennpunkt eines Parabolspiegels 16 in einem Gehäuse 17 angeordnet ist.

In Drehrichtung D den UV-Strahlungseinheiten 11, 12, 13, 14 nachgeordnet ist ein Abstreifer 18 vorgesehen, der dem Flüssigkeitsfilm, zum Beispiel den KSS-Film oder den Wasserfilm von der Tragfläche 5 abstreift. Die Flüssigkeit, zum Beispiel das KSS wird anschließend in einem nicht näher dargestellten Auffangbehälter gesammelt und dem FlüssigkeitsKreislauf wieder zugeführt.

Der Füllstand im Vorratsbehälter 8 bewegt sich im Betrieb im Bereich des Intervalls H, das der Höhe der Ausströmöffnung 10 entspricht. Der Vorratsbehälter 8 ist an seiner Unterseite 19 an die Kontur der Tragfläche 5 angepasst. Insbesondere im Bereich der Ausströmöffnung 10 liegt die Behälterwand 19 an der Tragfläche 5 an, so dass das durch die Ausströmöffnung 10 fließender KSS, Wasser und so weiter unmittelbar auf die Tragfläche 5 aufgetragen wird. Die obere Behälterwand 20 setzt sich in einem Fortsatz 21 parallel zur Tragfläche 5 fort und bildet somit einen Spalt 22, durch den der Flüssigkeitsfilm gefördert wird.

Die Drehgeschwindigkeit der Trägertrommel 3 bildet ebenso wie die Füllstandshöhe im Intervall H eine Stellgröße für die Mengenregelung der umgesetzten Flüssigkeit, zum Beispiel KSS. Je höher der Füllstand im Intervall H ausgebildet ist, desto dicker ist der durch den Spalt 22 geförderte, auf der Tragfläche 5 haftende Flüssigkeitsfilm. Je schneller die Trägertrommel 3 rotiert, umso größer ist die Abnahmemenge pro Zeiteinheit aus dem Vorratsbehälter 8.

Während der Drehung der Trägertrommel 3 wird der auf der Tragfläche 5 befindliche Flüssigkeitsfilm unter den UV-Strahlungseinheiten 11, 12, 13, 14 vorbeigeführt. Hierbei wird die Flüssigkeit, beispielsweise der KSS von dem UV-Licht der UV-Strahlungseinheiten 11, 12, 13, 14 durchstrahlt und dementsprechend entkeimt.

Durch die Anordnung der UV-Strahler 15 in den Brennpunkten der Parabolspiegel 16 wird die gesamte UV-Strahlung auf den Flüssigkeitsfilm gelenkt.

Die Tragfläche 5 kann in einer bevorzugten Ausführungsform als UV-Spiegel ausgebildet sein. In diesem Fall empfiehlt es sich, das Verhältnis zwischen der UV-Strahlungsintensität und der Filmdicke so auszugestalten, dass die UV-Strahlung mit ausreichender Intensität auf die als Reflexionsfläche ausgestaltete Tragfläche 5 trifft und von dort in den Flüssigkeitsfilm zurückreflektiert wird. Auf diese Weise wird eine homogenere Durchstrahlung des Flüssigkeitsfilms mit UV-Licht und somit eine homogenere Entkeimung bewirkt.

Die dargestellte Vorrichtung 1 stellt nur eine spezielle Ausführungsvariante der Erfindung dar. Wesentlich hierbei ist der Umstand, dass die Flüssigkeit, zum Beispiel der KSS als dünner Film ausgebildet wird, der von den UV-Strahlern 15 berührungslos zu durchstrahlen ist.

### Bezugszeichenliste:

- 1: Vorrichtung
- 2: Rahmengestell
- 3: Trägertrommel
- 4: Drehachse
- 5: Tragfläche
- 6: Randflansch
- 7: Gießkopf
- 8: Vorratsbehälter
- 9: Einfüllöffnung
- 10: Ausströmöffnung
- 11: UV-Strahlungseinheiten
- 12: UV-Strahlungseinheiten
- 13: UV-Strahlungseinheiten
- 14: UV-Strahlungseinheiten
- 15: UV-Strahler
- 16: Parabol-Spiegel
- 17: Gehäuse
- 18: Abstreifer
- 19: Behälterwand
- 20: Behälterwand
- 21: Fortsatz
- 22: Spalt

## Patentansprüche

1. Vorrichtung zur Behandlung von Flüssigkeiten, insbesondere Kühl- und Schmierstoffen (KSS) für den Einsatz bei der spanabhebenden Verarbeitung von Werkstoffen, mit einer Einrichtung zur Erzeugung eines Flüssigkeitsfilms, die einen als drehbare Trägertrommel (3) ausgebildeten Filmträger aufweist, mit einer UV-Strahlungseinheit zur berührungslosen Bestrahlung des Flüssigkeitsfilms und mit einem Abstreifer (18) zum Abtragen des Flüssigkeitsfilms von der Trägertrommel (3),
**dadurch gekennzeichnet, dass** ein Gießkopf (7) die Trägertrommel (3) in deren oberem Bereich anströmt, dass die UV-Strahlungseinheit (11, 12, 13, 14) dem Gießkopf (7) in Drehrichtung der Trägertrommel (3) nachgeordnet ist und die Trägertrommel (3) von außen bestrahlt und dass der Abstreifer (18) in Drehrichtung der Trägertrommel (3) der UV-Strahlungseinheit (11, 12, 13, 14) nachgeordnet ist.

2. Vorrichtung nach Anspruch 1,
**dadurch gekennzeichnet, dass** eine Mengenregulierung der umgesetzten Flüssigkeit durch eine Geschwindigkeitsregelung der Trägertrommel (3) vorgesehen ist.

3. Vorrichtung nach einem der vorgenannten Ansprüche,
**dadurch gekennzeichnet, dass** eine Mengenregulierung der umgesetzten Flüssigkeit durch eine Füllstandsregelung im Gießkopf (7) vorgesehen ist.

4. Vorrichtung nach einem der vorgenannten Ansprüche,
**dadurch gekennzeichnet, dass** die Trägertrommel (3) eine Oberflächenstruktur in der Tragfläche (5) aufweist.

5. Vorrichtung nach einem der vorgenannten Ansprüche,
**dadurch gekennzeichnet, dass** die Tragfläche (5) der Trägertrommel (3) als UV-Spiegel ausgebildet ist.

6. Vorrichtung nach einem der vorgenannten Ansprüche,
**dadurch gekennzeichnet, dass** die UV-Strahlungseinheit (11, 12, 13, 14) wenigstens eine Einheit mit einem optischen Element (16) zur Erhöhung der auf den Flüssigkeitsfilm eingestrahlten UV-Strahlung aufweist.

7. Vorrichtung nach einem der vorgenannten Ansprüche,
**dadurch gekennzeichnet, dass** die UV-Strahlungseinheit (11, 12, 13, 14) mehrere Einheiten aufweist.

## Claims

1. Device for the treatment of liquids, especially coolants and lubricants (KSS), for use in the machining of materials, with a mechanism for producing a liquid film, which mechanism comprises a film carrier formed as a rotatable carrier drum (3), with a UV radiation unit for noncontacting irradiation of the liquid film, and with a scraper (18) for removing the liquid film from the carrier drum (3),
**characterised in that** a pouring head (7) subjects the carrier drum (3) to a flow in the upper region thereof, that the UV radiation unit (11, 12, 13, 14) is disposed downstream of the pouring head (7) in the direction of rotation of the carrier drum (3) and irradiates the carrier drum (3) from outside, and that the scraper (18) is disposed downstream of the UV radiation unit (11, 12, 13 14) in the direction of rotation of the carrier drum (3).

2. Device according to Claim 1,
**characterised in that** the quantity of liquid which is displaced is regulated by regulating the speed of the carrier drum (3).

3. Device according to either of the preceding Claims,
**characterised in that** the quantity of liquid which is displaced is regulated by regulating the level in the pouring head (7).

4. Device according to any one of the preceding Claims,
**characterised in that** the carrier drum (3) has a surface structure in the carrying face (5).

5. Device according to any one of the preceding Claims,
**characterised in that** the carrying face (5) of the carrier drum (3) is formed as a UV reflector.

6. Device according to any one of the preceding Claims,
**characterised in that** the UV radiation unit (11, 12, 13, 14) comprises at least one unit with an optical element (16) for increasing the UV radiation which is radiated onto the liquid film.

7. Device according to any one of the preceding Claims,
**characterised in that** the UV radiation unit (11, 12, 13, 14) comprises a plurality of units.

## Revendications

1. Dispositif pour traiter des liquides, notamment des agents de refroidissement et des lubrifiants (KSS) applicables à l'usinage avec enlèvement de copeaux de matériaux, comportant une installation pour créer un film de liquide, cette installation comportant un support de film en forme de tambour de support (3), rotatif, une unité de rayonnement ultraviolet UV pour exposer sans contact le film liquide ainsi qu'un racloir (18) pour enlever le film liquide du support en forme de tambour (3),
**caractérisé en ce qu'**
une tête de coulée (7) alimente le tambour de support (3) dans sa partie supérieure, l'unité d'exposition aux rayons ultraviolets UV (11, 12, 13, 14) est prévue en aval de la tête de coulée (7) dans le sens de rotation du support en forme de tambour (3), le support en forme de tambour (3) est exposé de l'extérieur, et
le racloir (18) est en aval de l'unité d'exposition aux rayons ultraviolets (11, 12, 13, 14) dans le sens de rotation du support en forme de tambour (3).

2. Dispositif selon la revendication 1,
**caractérisé par**
une régulation de débit du liquide en circulation par une régulation de la vitesse du support en forme de tambour (3).

3. Dispositif selon l'une des revendications précédentes,
**caractérisé par**
une régulation de débit du liquide en circulation par une régulation de niveau de remplissage au niveau de la tête de coulée (7).

4. Dispositif selon l'une des revendications précédentes,
**caractérisé en ce que**
le support en forme de tambour (3) a une structure de surface dans la surface de support (5).

5. Dispositif selon l'une des revendications précédentes,
**caractérisé en ce que**
la surface de support (5) du support en forme de tambour (3) est un miroir réfléchissant le rayonnement ultraviolet.

6. Dispositif selon l'une des revendications précédentes,
**caractérisé en ce que**
l'unité d'exposition aux rayons ultraviolets UV (11, 12, 13, 14) comporte au moins une unité avec un élément optique (16) pour augmenter le rayonnement ultraviolet UV traitant le film de liquide.

7. Dispositif selon l'une des revendications précédentes,
**caractérisé en ce que**
l'unité d'exposition aux rayonnements ultraviolets UV (11, 12, 13, 14) comporte plusieurs éléments.
